(19) Europäisches Patentamt
European Patent Office
Office européen des brevets

(11) Publication number: **0 048 043**
**A1**

(12) # EUROPEAN PATENT APPLICATION

(21) Application number: 81200927.2

(22) Date of filing: 25.08.81

(51) Int. Cl.³: A 61 K 31/495
C 07 D 295/12

(30) Priority: 12.09.80 NL 8005131

(43) Date of publication of application: 24.03.82 Bulletin 82/12

(84) Designated Contracting States: BE CH DE FR GB IT LI LU NL SE

(71) Applicant: DUPHAR INTERNATIONAL RESEARCH B.V
C.J. van Houtenlaan 36
1381 CP Weesp(NL)

(72) Inventor: Haeck, Hans Heinz
c/o OCTROOIBUREAU ZOAN B.V. Apollolaan 151
NL-1077 AR Amsterdam(NL)

(72) Inventor: Hillen, Feddo Cornelius
c/o OCTROOIBUREAU ZOAN B.V. Apollolaan 151
NL-1077 AR Amsterdam(NL)

(74) Representative: Muis, Maarten, Drs. et al,
OCTROOIBUREAU ZOAN B.V. Apollolaan 151
NL-1077 AR Amsterdam(NL)

(54) **Phenyl piperazine derivatives having analgetic activity.**

(57) It has been found that the group of phenyl piperazine derivatives of formula 3 of the formula sheet and the salts thereof have a strong analgetic activity. The compounds can be prepared in a manner known for analogous compounds and be processed to the usual compositions.

EP 0 048 043 A1

Croydon Printing Company Ltd.

Phenyl piperazine derivatives having analgetic activity.

The invention relates to phenyl piperazine derivatives and to compositions comprising at least one of these compounds as an active substance.

Various phenyl piperazine derivatives having varying pharmacological properties are known.

For example, United States Patent Specification 2,722,529 relates to compounds of formula 1 of the formula sheet, in which Y is a straight or a branched alkyl group having 2-6 carbon atoms and Z is an acyl group, a sulphonyl group or a carbamoyl group. These compounds are said to have a strong sympatholytic activity and sometimes a hypotensive activity.

Furthermore, United States Patent Specification 2,833,770 discloses a group of compounds of the general formula 2 of the formula sheet, in which formula R is a chlorine atom or a bromine atom, R' is hydrogen or a methyl group, and n is an integer from 2-5. Of these compounds also a sympatholytic activity is known.

Non-pre-published European Patent Application 80200173.5 relates to a new group of phenyl piperazine derivatives which have a strong antiagressive activity.

It has been found that phenyl piperazine derivatives of formula 3 of the formula sheet, in which $R_1$ is a hydrogen atom, a methyl group, a methoxy group or an amino group, $R_2$ is a hydrogen atom or a methyl group and $R_3$ is a trifluoromethyl group or a chlorine atom, and the salts thereof, have a strong analgetic activity. This group of compounds belongs to the group of phenyl piperazine derivatives described in the above-mentioned European Patent Application.

Analgetically active compounds according to the invention are, for example:

1-[2-(N-acetylamino)ethyl]-4-(3-chlorophenyl)piperazine, 1-[2-(N-acetylamino)ethyl]-4-(3-trifluoromethylphenyl)piperazine,

1-[2-(N-aminocarbonylamino)ethyl]-4-(3-chlorophenyl)piperazine, 1-[2-(N-aminocarbonylamino)ethyl]-4-(3-trifluoromethylphenyl)piperazine,

1-[2-(N-acetyl-N-methylamino)ethyl]-4-(3-trifluoromethylphenyl)piperazine,

1-[2-(N-formylamino)ethyl]-4-(3-chlorophenyl)piperazine, 1-[2-(N-formylamino)ethyl]-4-(3-trifluoromethylphenyl)piperazine, and

1-[2-(N-methoxycarbonylamino)ethyl]-4-(3-trifluoromethylphenyl)piperazine.

Surprisingly, the said analgetic activity of the compounds according to the invention has been found not to be associated with the sympatholytic properties described for the above-mentioned known and structurally closely related compounds, which properties are undesired for analgetics.

Other side effects undesired for use as analgetics, for example, dopaminolytic properties, muscle relaxing properties and sedative properties, are also absent in the dosages in which the analgetic activity occurs.

It is surprising that the compounds according to the invention have such a strong analgetic activity, since structurally closely related known compounds have quite a different pharmacological activity pattern.

The analgetically active compounds according to the invention have $ED_{50}$-values which vary from 4 to 10 mg/kg, while structurally related compounds known from the above-mentioned United States Patent Specification generally show $ED_{50}$-values of more than 100 mg/kg. These $ED_{50}$-values were determined in an analgetic test in mice (Brit. J. Pharm. 9, (1954), 280). In this test the pain stimulus was generated by placing bulldog clips on the tailhead of a mouse. The animals try to remove the pain stimulus by biting. The non-occurrence of the pain response after administration of test substances is a measure of the analgetic activity. The compounds to be tested were administered orally. Five mice were used per dose. Sixty minutes after the administration of the compounds to be tested, the occurrence of the pain response was established. From the results obtained the $ED_{50}$-values in mg/kg of active substance per kg of body weight were calculated. Since in the compounds no undesired side effects were found, these readily analgetically active substances are suitable for use in humane medicine in the treatment of both acute pain and chronic pain.
The quantity, frequency and way of administration may differ for each individual case, also dependent on the nature and the severity of the disturbances. For use in men a dosing of 5-500 mg and preferably from 25-150 mg daily will generally be suitable.

The active compounds according to the invention and their salts can be processed to compositions such as pills, tablets, coated tablets, capsules, powders, injection liquids and the like according to the known standard methods while using the conventional auxiliary substances such as solid and liquid carrier materials.

As examples of pharmaceutically acceptable acids with which the compounds according to the invention can form salts may be mentioned hydrochloric acid, sulphuric acid, nitric acid, citric acid, fumaric acid, maleic acid, tartaric acid, methanesulfonic acid, benzoic acid, and the like.

The compounds of formula 3 and their salts can be prepared according to the methods which are suitable for the synthesis of analogous compounds. Suitable preparation methods are described in the above-mentioned European Patent Application 80200173.5.

EXAMPLE I

Tablet:

50 mg of 1-[2-(N-aminocarbonylamino)ethyl]-4-(3-trifluoromethylphenyl)piperazine.HCl
335 mg of lactose
60 mg of potato starch
25 mg of talcum
5 mg of magnesium stearate
5 mg of gelatin

EXAMPLE II

Suppository:

50 mg of 1-[2-(N-aminocarbonylamino)ethyl]-4-(3-trifluoromethylphenyl)piperazine.HCl
1500 mg of suppository mass

EXAMPLE III

Injection liquid:

25 g of 1-[2-(N-aminocarbonylamino)ethyl]-4-(3-trifluoromethylphenyl)piperazine.HCl
1.80 g of methyl p-hydroxybenzoate
0.20 g of propyl p-hydroxybenzoate
9.0 g of sodium chloride
26 g of glycerin
1 g of benzyl alcohol
water up to 1,000 g.

CLAIMS:

1. Pharmaceutical compositions, characterized in that they comprise as an active substance at least one compound of formula 3 of the formula sheet, in which:

$R_1$ is a hydrogen atom, a methyl group, a methoxy group or an amino group,

$R_2$ is a hydrogen atom or a methyl group, and

$R_3$ is a chlorine atom or a trifluoromethyl group,

or a pharmaceutically acceptable acid addition salt thereof.

2. A method of preparing pharmaceutical compositions, characterized in that a compound of formula 3, in which $R_1$, $R_2$ and $R_3$ have the meanings mentioned in Claim 1, or a pharmaceutically acceptable acid addition salt thereof, is brought into a form suitable for administration.

3 . A method of controlling pain in man or animal, characterized in that an active quantity of a compound of formula 3, in which $R_1$, $R_2$ and $R_3$ have the meanings given in Claim 1, or a salt thereof with a pharmaceutically acceptable acid, is administered.

DIR 0313

1/1

0048043

FORMULA-SHEET

1. 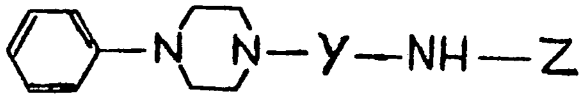

2. 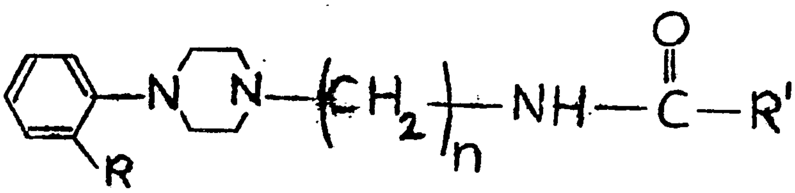

3. 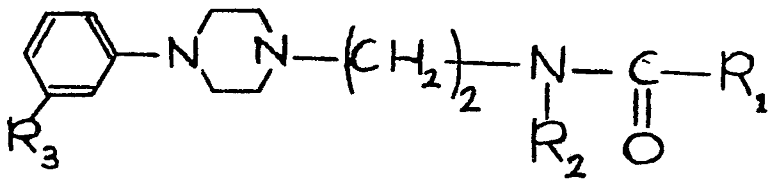

DUPHAR INTERNATIONAL RESEARCH B.V.

0048043
Application number

European Patent Office

# PARTIAL EUROPEAN SEARCH REPORT

which under Rule 45 of the European Patent Convention shall be considered, for the purposes of subsequent proceedings, as the European search report

EP 81 20 0927

## DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| X | AU - A - 55848/80 (DUPHAR)<br>* Claims *<br>& EP - A - 0 015 615<br>-- | 1,2 | A 61 K 31/495<br>C 07 D 295/12 |
| | GB - A - 1 368 256 (PFIZER)<br>* Claims *<br>---- | 1,2 | |

TECHNICAL FIELDS SEARCHED (Int. Cl.³)

C 07 D 295/12
B 61 K 31/495

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2
Claims searched incompletely: 3
Claims not searched:
Reason for the limitation of the search: Method for treatment of the human or animal body by therapy (see art. 52(4) of the European Patent Convention).

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| Place of search | Date of completion of the search | Examiner |
|---|---|---|
| The Hague | 17-12-1981 | MOREAU |

EPO Form 1505.1 06.78